# EUROPEAN PATENT SPECIFICATION

(11) **EP 1 870 827 B1**
(45) Date of publication and mention of the grant of the patent: **14.04.2010**
(21) Application number: 07011870.8
(22) Date of filing: 18.06.2007
(51) Int. Cl.: G06F 19/00, A61B 19/00

(54) **Technique image recording control system, technique image recording control method and operation system**
Steuerungssystem für eine Bildaufzeichnung eines Verfahrens, zugehörige Verfahren und Betriebssystem
Système de commande de l'enregistrement d'images d'une opération technique, sa méthode et son système opératif

(30) Priority: 21.06.2006 JP 2006171771
(43) Date of publication of application: 26.12.2007
(73) Proprietor: OLYMPUS MEDICAL SYSTEMS CORP., Tokyo (JP)
(72) Inventor: Tashiro, Koichi, Tokyo 151-0072 (JP)
(74) Representative: von Hellfeld, Axel

(56) References cited:
- WO-A-2005/102142
- JP-A- 2002 233 535
- US-A1- 2003 078 812
- US-A1- 2004 204 627
- US-A1- 2005 182 296
- US-A1- 2005 234 326
- US-A1- 2005 283 138
- US-A1- 2005 284 491

## Description

### BACKGROUND OF THE INVENTION

### 1. Field of the Invention

The present invention relates to a technique image recording control system, technique image recording control method and operation system that control recording multiple kinds of images picked up during a technique.

### 2. Description of the Related Art

In recent years, an endoscopic operation system that performs a technique by using an endoscope has been widely spread, and wide varieties of medical appliance are used therein.

Medical appliances used in the endoscopic operation system include an electric knife device, an ultrasonic device and an insufflator in addition to an electronic endoscopic system. As disclosed in Japanese Unexamined Patent Application Publication No. 2003-76786 or 2003-70746, for example, these appliances are centrally managed in a system and are controlled by an operation device deployed under a system controller.

On the other hand, various peripheral appliances in addition to the medical appliances above are placed in an operation room having a medical system, typified by the endoscopic operation system. For example, the peripheral appliances may include a server that stores a reference image such as a CT image, and an ultrasonic image before an operation of a patient, a display device that displays the reference image, which is referred for a technique, an output device that records or prints an endoscopic image picked up by the endoscopic operation system, a video conference system that communicates with the outside of the operation room, and a room light that controls the illumination in the operation room.

For the variety of peripheral appliances, an AV system is established, which is a non-medical appliance system separately provided from a system that controls a medical appliance and is controlled by a different operation device from the operation device for controlling a medical appliance. For example, in an operation room, operational records are saved in images by the AV system for appliance management and/or with the introduction of Board Certification.

However, images to be stored have different degrees of importance depending on the progress of an operation. For example, an endoscopic image is more important during a laparoscopic operation while an image of an environment in an operation room (that is, room camera image, for example) and/or an image of the hand of a doctor and surroundings (that is, ceiling image, for example) are more important before a laparoscope is used though an endoscopic image is not needed. In this way, the images to be recorded have different degrees of importance depending on the progress of an operation.

Even a necessary image required to record accurately may not be recorded in a desired recorder in a conventional endoscopic operation system or AV system that instructs image recording manually by simply forgetting the pickup (or forgetting recording the image), missing the timing for switching and outputting an image to a desired recorder due to other operations, performing improper switching among recorders.

US 2005/0284491 A1 discloses an operating room control system with a first controller connected to a medical device and a second controller connected to a nonmedical device. There is also provided an operating instruction input device accepting input for the medical device and also transmitting input to the second controller connected to a nonmedical device based on instructions entered into the operating instruction input device.

US 2005/0283138 A1 discloses a control system with a system controller connected to medical devices and an AV-controller connected to audio-visual equipment. There is also provided a switcher providing different control interfaces on a screen depending on a context of operation.

Documents US 2005/0234326 A1, US 2005/0182296 A1, JP 2002-233535 and WO 2005/102142 A1 also refer to medical system control devices comprising at least one image recording means.

### SUMMARY OF THE INVENTION

Accordingly, the invention was made in view of these points, and it is an object of the present invention to provide an operation system that can securely record an optimum technique image in accordance with the progress of an operation.

According to the present invention, there is provided an operating system according to claim 1 and a method according to claim 6.

The other features and advantages of the present invention will be sufficiently apparent from following descriptions.

### BRIEF DESCRIPTION OF THE DRAWINGS

Fig. 1 is a configuration diagram showing a configuration of an endoscopic operation system according to a first embodiment of the present invention;
Fig. 2 is a block diagram showing a configuration of an operation room control system that has the endoscopic operation system in Fig. 1 according to the first embodiment;
Fig. 3 is a block diagram showing a configuration of the system controller in Fig. 2 according to the first embodiment;
Fig. 4 is a block diagram showing a configuration of the AV controller in Fig. 2 according to the first embodiment;
Fig. 5 is a diagram illustrating a control trigger signal generated by the control trigger signal generating section in Fig. 3 according to the first embodiment;
Fig. 6 is a flowchart showing a flow of processing by the endoscopic operation system in Fig. 2 according to the first embodiment;
Fig. 7 is a flowchart showing a flow of processing by the AV system in Fig. 2 according to the first embodiment;
Fig. 8 is an explanatory diagram on a specific example of the processing in Figs. 6 and 7 according to the first embodiment;
Fig. 9 is a first diagram showing an example of a screen to be displayed on the operation panel in Fig. 2 according to the first embodiment;
Fig. 10 is a second diagram showing another example of the screen to be displayed on the operation panel in Fig. 2 according to the first embodiment;
Fg. 11 is a third diagram showing another example of the screen to be displayed on the operation panel in Fig. 2 according to the first embodiment;
Fig. 12 is a block diagram showing a configuration of a first variation example of the operation room control system in Fig. 2 according to the first embodiment;
Fig. 13 is a block diagram showing a configuration of a second variation example of the operation room control system in Fig. 2 according to the first embodiment;
Fig. 14 is a block diagram showing a configuration of an operation room control system according to a second embodiment of the present invention;
Fig. 15 is a block diagram showing a configuration of the AV controller in Fig. 14 according to the second embodiment;
Fig. 16 is a first diagram showing an example of a synthesized image resulting from the synthesis in the image synthesizing device in Fig. 14 according to the second embodiment;
Fig. 17 is a second diagram showing another example of a synthesized image resulting from the synthesis in the image synthesizing device in Fig. 14 according to the second embodiment;
Fig. 18 is a first diagram showing an example of the screen to be displayed on the operation panel in Fig. 14 according to the second embodiment;
Fig. 19 is a second diagram showing another example of the screen to be displayed on the operation panel in Fig. 14 according to the second embodiment;
Fig. 20 is a block diagram showing a configuration of a variation example of the operation room control system in Fig. 20 according to the second embodiment;
Fig. 21 is a block diagram showing a configuration of an operation room control system according to a third embodiment of the present invention;
Fig. 22 is a first diagram showing an example of the screen to be displayed on the operation panel in Fig. 21 according to the third embodiment;
Fig. 23 s a second diagram showing another example of the screen to be displayed on the operation panel in Fig. 21 according to the third embodiment; and
Fig. 24 is a third diagram showing another example of the screen to be displayed on the operation panel in Fig. 21 according to the third embodiment.

### DETAILED DESCRIPTION OF THE PREFERRED EMBODIMENTS

### [First Embodiment]

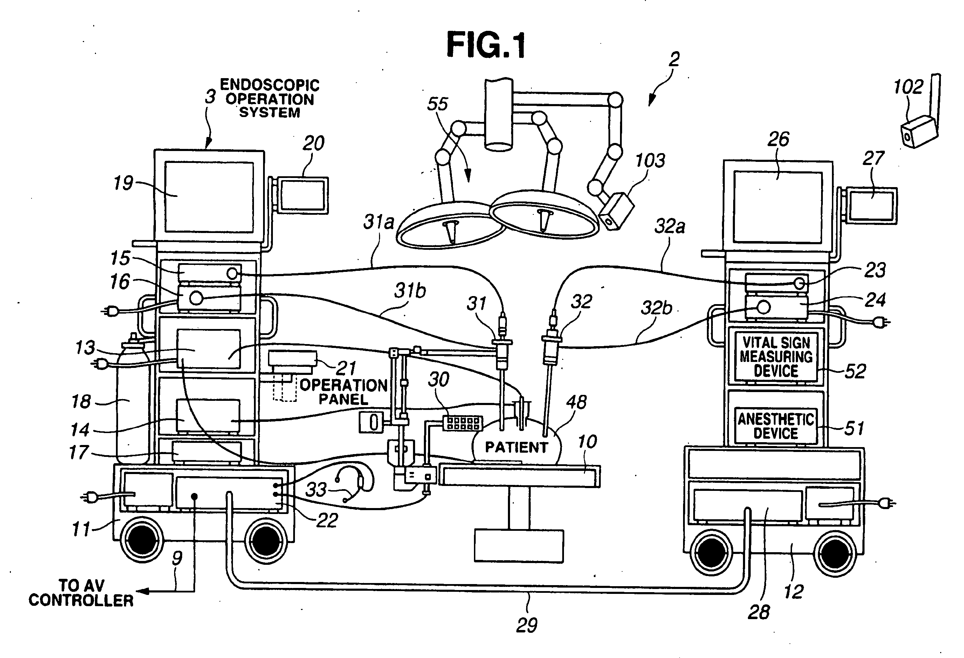

With reference to Fig. 1, an entire configuration of an endoscopic operation system 3 functioning as a first control system placed in an operation room 2 will be described first.

As shown in Fig. 1, a patient bed 10 on which a patient 48 lies down and the endoscopic operation system 3 are placed within the operation room 2. The endoscopic operation system 3 has a first cart 11 and a second cart 12. A shadowless lamp 55 illuminating a part at the hand of an operating surgery is provided on the ceiling above the patient bed 10. A ceiling camera 103 is placed near the shadowless lamp 55, and the ceiling camera 103 picks up an image of a part at the hand of an operating surgeon as required. A room camera 102 is also placed on the ceiling of the operation room 2. The room camera 102 picks up an image of an entire view of the operation room 2 during an operation.

The first cart 11 may have, for example, an electric knife device 13, an insufflator 14, an endoscope camera device 15, a light source device 16 and a gas cylinder 18 filled with carbon dioxide, which are medical appliances to be controlled. The endoscope camera device 15 is connected to a first endoscope 31 via a camera cable 31a. The light source device 16 is connected to the first endoscope 31 via a light guide cable 31b.

The first cart 11 further has a display device 19, a first central display panel 20, an operation panel 21 functioning as output image display means. The display device 19 may display an endoscopic image from the endoscope camera device 15. For example, the display device 19 may be a TV monitor. The central display panel 20 functions as display means that can selectively display any data during an operation. The operation panel 21 includes a display section such as a liquid crystal display and a touch panel, for example, integrated onto the display section and functions as a central operation device to be operated by a nurse in a non-clean area (non-disinfected area).

The first cart 11 further has a system controller 22, which functions as a control device. The system controller 22 is connected to the electric knife device 13, insufflator 14, endoscope camera device 15 and light source device 16 through a communication line, not shown, and controls these appliances through serial communication. A head-set type microcomputer 33 can be connected to the system controller 22. The system controller 22 recognizes voice inputted from the microcomputer 33 and can control each appliance by voice of a surgeon.

On the other hand, the second cart 12 has an endoscope camera device 23, a light source device 24, an anesthetic device 51, a vital sign measuring device 52, a display device 26 and a second central display panel 27, which are to be controlled. The endoscope camera device 23 is connected to a second endoscope 32 via a camera cable 32a. The light source device 24 is connected to the second endoscope 32 via a light guide cable 32b. The display device 26 may display an endoscopic image captured by the endoscope camera device 23. The second central display panel 27 can selectively display any data during an operation.

The endoscope camera device 23, light source device 24 and an image processing device 25 are connected to a relay unit 28 in the second cart 12 through a communication line, not shown. Then, the relay unit 28 is connected to the system controller 22 in the first cart 11 through the relay cable 29.

Thus, the system controller 22 can centrally control the camera device 23, light source device 24 and anesthetic device 51 in the second cart 12 and the electric knife device 13, insufflator 14, camera device 15 and light source device 16 in the first cart 11.

Therefore, when the system controller 22 communicates with these devices, the system controller 22 can display a setting state of the connected appliances and/or a setting screen for an operation switch, for example, on the liquid crystal display of the operation panel 21 above.

The system controller 22 allows an operation input for changing a set value, for example, by touching a desired operation switch on the operation panel 21 and operating a touch panel in a predetermined area.

The remote controller 30 is a second central operation device to be operated by a surgeon in a clean area (disinfected area) and allows other devices the connection with which is established to be operated through the system controller 22.

The system controller 22 is connected to the vital sign measuring device 52 through the relay unit 28 and can analyze biological information obtained from the vital sign measuring device 52 and display the analysis result on a desired display device.

The system controller 22 is further connected to the anesthetic device 51 through the relay unit 28 and can monitor the anesthetic state of the patient 48 by the anesthetic device 51 and display the monitoring result on a desired display device.

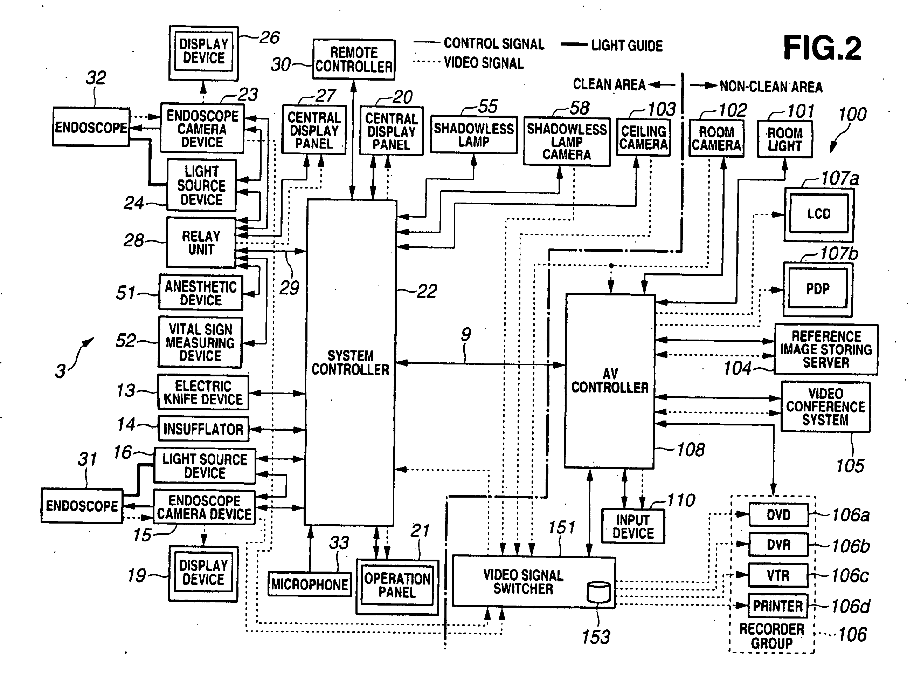

In the operation room 2, an operation room control system, as shown in Fig. 2, is established which has the endoscopic operation system 3 (refer to Fig. 1). In other words, the operation room control system includes the endoscopic operation system 3 and an AV system 100 functioning as a second control system having various audio visual appliances and illumination appliances.

The AV system 100 includes a room light 101 that illuminates the entire operation room 2, a room camera 102 that picks up an image of the entire operation room 2, a server 104 storing reference images, a video conference system 105, a recorder group 106, an LCD 107a, a PDP 107b, an input device 110, a video signal switcher 151 functioning as image output means and an AV controller 108 that controls these devices.

The reference images storing server 104 stores a CT image and/or ultrasonic image of the patient 48 before an operation, and these images can be displayed on the LCD 107a and/or PDP 107b.

The input device 110 includes a pointing device by a GUI (graphic user interface) for a touch panel, for example. The input device 110 may include a mouse and/or a keyboard.

The AV controller 108 controls the video signal switcher 151. The video signal switcher 151 receives the input of video signals from the endoscope camera devices 15 and 23 of the endoscopic operation system 3 and ceiling camera 103 and video signals from the room camera 102 of the AV system 100. The video signal switcher 151 selectively outputs video signals inputted under the control of the AV controller 108 to a DVD 106a, a DVR 106b, a VTR 106c and a printer 106d included in the recorder group 106. Thus, the AV controller 108 controls to record video signals by outputting video signals selectively to the DVD 106a, DVR 106b, VTR 106c and printer 106d.

The video signal switcher 151 also outputs video signals outputted to the recorder group 106 to the system controller 22. The system controller 22 displays the video signals from the video signal switcher 151 on the operation panel 21 so that a surgeon can monitor the image (video signals) outputted to the recorder group 106. Furthermore, the video signal switcher 151 has a video storage section 153 that updates and records the latest video signals in five minutes, for example, of each inputted video signal as a frame image.

Here, the system controller 22 and the AV controller 108 exchange information by a serial communication protocol, for example, via a cable 9 and can monitor the mutual control states by each other.

The shadowless lamp 55 may have a shadowless lamp camera 58. If the shadowless lamp camera 58 is installed in the shadowless lamp 55, video signals from the shadowless lamp camera 58 may be configured to input to the video signal switcher 151.

Having shown the operation panel 21 in the clean area in Fig. 2, the operation panel 21 is placed in the non-clean area as described above.

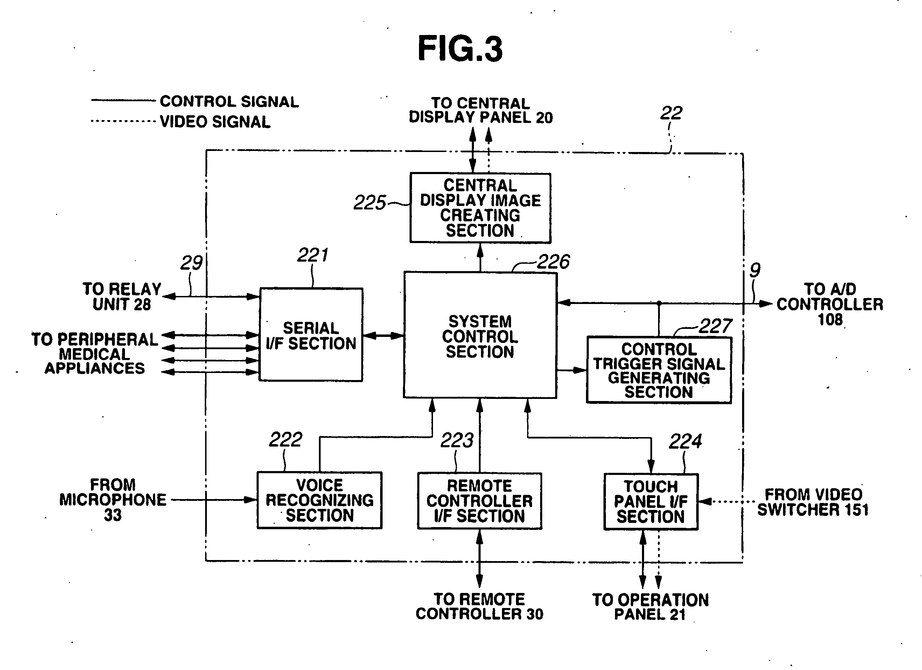

The system controller 22 includes, as shown in Fig. 3, a serial I/F (interface) section 221, a voice recognizing section 222, a remote controller I/F section 223, a touch panel I/F section 224, a central display image creating section 225, a system control section 226 functioning as appliance monitoring means and a control trigger signal generating section 227 functioning as control signal generating means.

The serial I/F section 221 is an I/F section that performs serial communication such as RS-232 with peripheral medical appliances such as the electric knife device 13, insufflator 14, endoscope camera device 15 and light source device 16 in the first cart 11 and the relay unit 28 in the second cart 12. Through the relay unit 28, the serial I/F section 221 can perform data communication with the endoscope camera device 23, light source device 24, anesthetic device 51 and vital sign measuring device 52, which are peripheral medical appliances in the second cart 12.

The voice recognizing section 222 samples voice signals from the microphone 33, recognizes them as voice data and outputs the recognized voice data to the system control section 226 as character data.

The remote controller I/F section 223 is an I/F section that outputs data from a remote controller to the system control section 226.

The touch panel I/F section 224 is an I/F section for implementing a touch-panel function of the operation panel 21. In other words, the touch panel I/F section 224 transmits a touched image created by the system control section 226 to the operation panel 21, receives the input of a coordinate signal thereof from the operation panel 21 and outputs the coordinate signal to the system control section 226.

The touch panel I/F section 224 receives the input of video signals from the video signal switcher 151, and displaying the video signals on the operation panel 21 as described above allows a surgeon to monitor the image (video signal) outputted to the recorder group 106.

The central display image creating section 225 is an image creating section that creates a display image in accordance with control information on a peripheral medical appliance, which is inputted through the serial I/F section 221 and is to be displayed by the central display panel 20, by capturing the control information from the system control section 226.

When the occurrence of an event for recording control that instructs to start/stop recording an image of a peripheral medical appliance is detected by the system control section 226 through the serial I/F section 221, the control trigger signal generating section 227 in response to the event occurrence generates a control trigger signal, which will be described later, and outputs the control trigger signal to the AV controller 108 via the cable 9.

The details of the event occurrence for recording control that instructs to start/stop recording an image will be described later.

The system control section 226 is a control section that controls the components described above of the system controller 22.

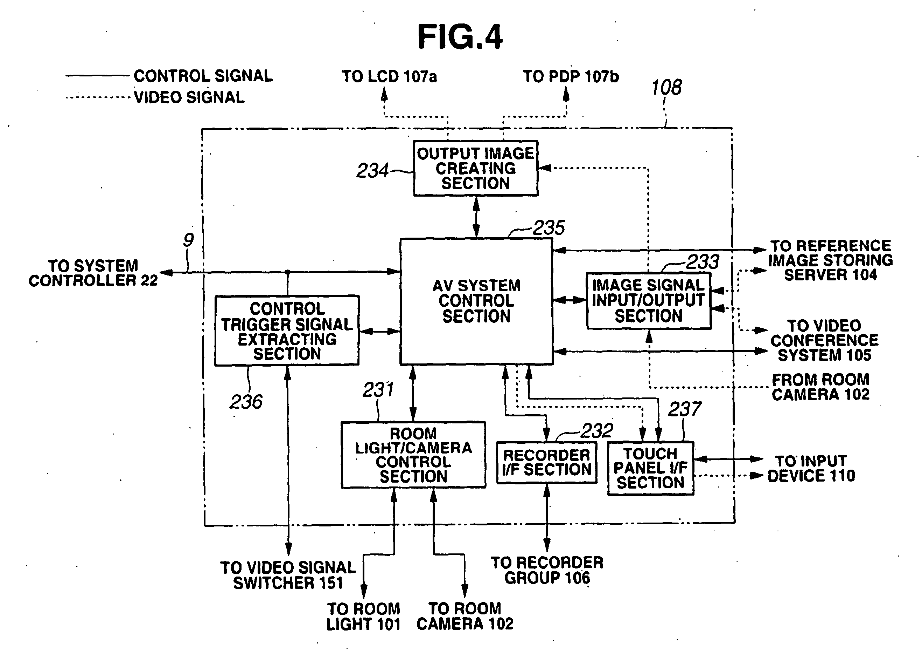

The AV controller 108 includes, as shown in Fig. 4, a room light/camera control section 231, a recorder I/F section 232, an image signal input/output section 233, an output image creating section 234, an AV system control section 235, a control trigger signal extracting section 236 and a touch panel I/F, section 237.

The image recording control means includes the AV system control section 235 and the control trigger signal extracting section 236.

The touch panel I/F section 237 is an I/F section for implementing a touch panel function of the input device 110. The room light/camera control section 231 is a control section that controls the lighting up of the room light 101 and the driving of the room camera 102 based on an instruction from the input device 110. The recorder I/F section 232 is an I/F section that communicates with the DVD 106a, DVR 106b, VTR 106c, and printer 106d in the recorder group 106.

The image signal input/output section 233 is an input/output section that receives the input of video signals from the room camera 102, video conference system 105 and reference image storing server 104 and outputs the video signals to the output image creating section 234 and outputs the video signals from the room camera to the video conference system 105 and reference image storing server 104. The output image creating section 234 is an image creating section that creates a display image to be displayed on the LCD 107a and PDP 107b from the video signals from the image signal input/output section 233.

The control trigger signal extracting section 236 is a signal extracting section that extracts a control trigger signal from the system controller 22 and outputs the control trigger signal to the AV system control section 235.

The AV system control section 235 is a control section that controls the components above and controls the video signal switcher 151 through the control trigger signal extracting section 236.

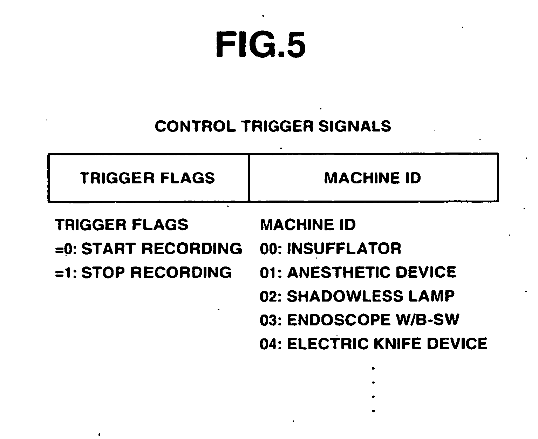

Next, the control trigger signal will be described which is generated by the control trigger signal generating section 227 in the system controller 22 (refer to Fig. 3). In response to the detection of the occurrence of an event in a peripheral medical appliance through the serial I/F section 221, the control trigger signal generating section 227 generates a control trigger signal including a trigger flag and an appliance ID, as shown in Fig. 5, in accordance with the state of the event.

The trigger flag of the control trigger signal is a flag that instructs starting or stopping recording to one appliance of the DVD 106a, DVR 106b, VTR 106c and printer 106d in the recorder group 106. For example, the trigger flag = 0 means to start recording while the trigger flag = 1 means to stop recording.

The appliance ID of the control trigger signal is identification information for identifying a medical appliance in which an event occurs. For example, Appliance ID = 00 is identification information of the insufflator 14, Appliance ID = 01 is identification information of the anesthetic device 51, Appliance ID = 02 is identification information of the shadowless lamp 55, Appliance ID = 03 is identification information of a W/B (white balance) -SW (not shown) of the endoscope 31, Appliance ID = 04 is identification information of the electric knife device 13, and so on.

In response to a control trigger signal through the control trigger signal extracting section 236, the AV system control section 235 selects a recorder which corresponds to the appliance ID in advance within the recorder group 106 and controls the video signal switcher 151 through the control trigger signal extracting section 236 to start/stop recording to the recorder corresponding to the appliance ID based on the trigger flag.

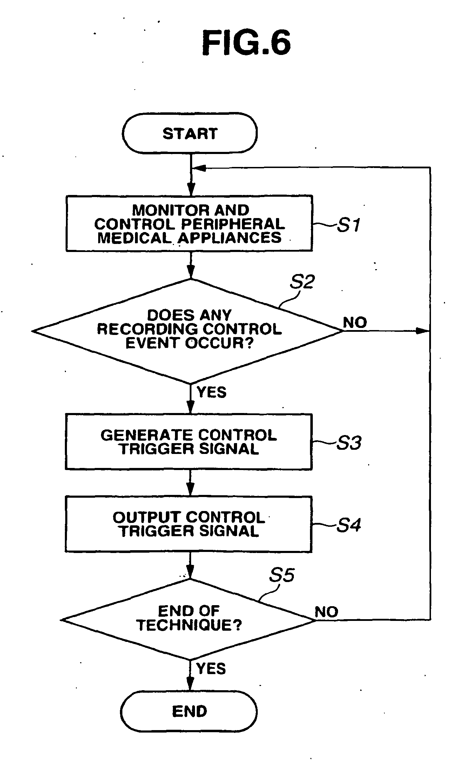

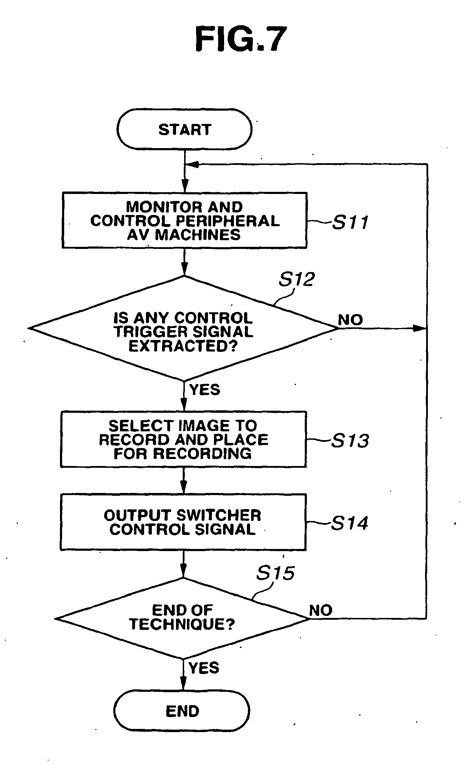

Operations of the present embodiment having this configuration will be described with reference to the flowcharts in Figs. 6 and 7.

When a technique is started and the system controller 22 is powered on, the system controller 22 starts monitoring and controlling the peripheral medical appliances connected through the serial I/F section 221 in step S1, as shown in Fig. 6. Then, the system controller 22 waits for the determination whether or not an event has occurred for recording control that instructs the peripheral medical appliances to start/stop image recording in step S2.

The word, "event", here refers to a change in control states of the medical appliances registered within the system controller 22 in advance. For example, when the system controller 22 detects the start or stop of the anesthetic device 51, the start or stop of the insufflator 14, the output of a high frequency output of the electric knife device 13, the switch on/off of the shadowless lamp 55, the switch operation(s) on the endoscopes 31 and/or 32, and/or a switch operation on an endoscope camera device, the system controller 22 determines that the event has occurred.

Upon detection of the occurrence of such an event, the system controller 22 in step S3 sets a trigger flag in accordance with operational states of the peripheral medical appliances in which the events occur and causes the control trigger signal generating section 227 to generate a control trigger signal having a combination of appliance IDs of the peripheral medical appliances in which the events occur by a predetermined protocol (which is a protocol by which the system controller 22 and the AV controller 108 can communicate via the cable 9 and will be simply called "predetermined protocol" below).

For example, when the control over the insufflator 14 starts, the system controller 22 detects the control start signal as an event that instructs to start image recording in the insufflator 14. Then, the system controller 22 outputs data including Trigger Flag = 0 and Appliance ID = 00 (refer to Fig. 5) to the control trigger signal generating section 227. The control trigger signal generating section 227 generates a control trigger signal by the predetermined protocol from the data.

On the other hand, when the control over the insufflator 14 stops, for example, the system controller 22 detects the control stop signal as an event that instructs to stop image recording in the insufflator 14. Then, the system controller 22 outputs data including Trigger Flag = 1 and Appliance ID = 00 (refer to Fig. 5) to the control trigger signal generating section 227. The control trigger signal generating section 227 generates a control trigger signal by the predetermined protocol from the data.

Next, the system controller 22 in step 54 causes the control trigger signal generating section 227 to output the control trigger signal by the predetermined protocol to the AV controller 108 via the cable 9 and repeats the processing in steps S1 to S5 above until the detection of the end of the technique in step S5.

On the other hand, when a technique starts and the AV controller 108 is powered on, the AV controller 108 in step S11 starts monitoring and controlling the peripheral AV appliances (including the room light 101, room camera 102, reference image storing server 104, video conference system 105, recorder group 106, LCD 107a and PDP 107b) through the room light/camera control section 231, recorder I/F section 232, image signal input/output section 233 and output image creating section 234, as shown in Fig. 7.

Then, the AV controller 108 in step S12 waits for the determination on whether a control trigger signal has been extracted from the communication data from the system controller 22 by the control trigger signal extracting section 226 or not.

If the control trigger signal is extracted, the AV controller 108 in step S13 selects an image to record and an appliance for recording based on the control trigger signal.

Here, the AV system control section 235 of the AC controller 108 internally stores as table data the image to record and an appliance for recording, which are associated with the appliance ID of the control trigger signal, such as:
Appliance ID = 00 (insufflator): image to record = endoscopic image and appliance for recording = DVD 106a,
Appliance ID = 01 (anesthetic device): image to record = room camera image and appliance for recording = VTR 106b, and
Appliance ID = 02 (shadowless lamp): image to record = ceiling camera image and appliance for recording = DVR 106c.

The AV controller 108 in step S 14 outputs a switcher control signal to the video signal switcher 151. The video signal switcher 151 uses the switcher control signal to control the video output of the image to record associated in step S 13 to the appliance for recording, which is also associated, based on the trigger flag.

Fig, 8 shows a change in control to start/stop recording video signals (image to record) to the destination (appliance for recording) when an event occurs in the appliance(s), which corresponds to the progress of an operation where the appliance(s) in which the event occurs is/(are) referred by Appliance ID = 00 (insufflator), Appliance ID = 01 (anesthetic device) and/or Appliance ID = 02 (shadowless lamp).

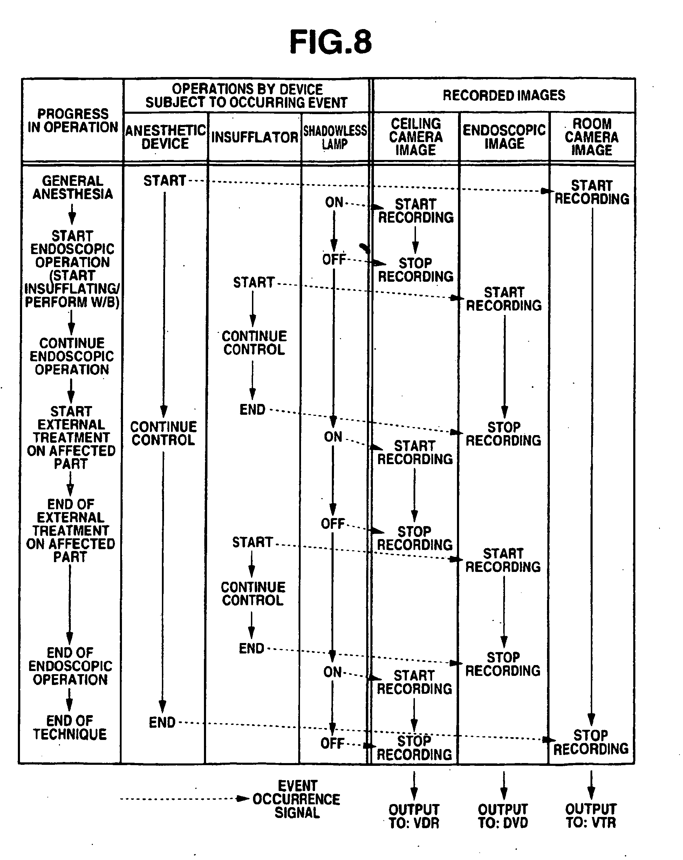

More specifically, as shown in Fig. 8, when a technique starts and the system controller 22 starts controlling the anesthetic device 51 for performing general anesthesia on the patient 48, the system controller 22 detects the event occurrence of the control start by the anesthetic device 51.

By detecting the event occurrence by the system controller 22, a control trigger signal = {Trigger Flag = 0 (=start recording), Appliance ID = 01} is transmitted from the system controller 22 to the AV controller 108.

Then, based on the control trigger signal = {Trigger Flag = 0 (=start recording), Appliance ID = 01}, the AV controller 108 controls the VTR 106c through the recorder I/F section 232 and controls the video signal switcher 151 through the control trigger extracting section 236 to select video signals from the room camera 102 and select the VTR 106c as the destination and start recording a room camera image in the VTR 106c.

When a technique is started, the controller 22 switches on the shadowless lamp 55. As a result, the system controller 22 detects the occurrence of the switch-on (ON) event from the shadowless lamp 55.

By detecting the event occurrence by the system controller 22, a control trigger signal = {Trigger Flag = 0 (=start recording), Appliance ID = 02} is transmitted from the system controller 22 to the AV controller 108.

Then, based on the control trigger signal = {Trigger Flag = 0 (=start recording), Appliance ID = 02}, the AV controller 108 controls the DVR 106b through the recorder I/F section 232 and controls the video signal switcher 151 through the control trigger extracting section 236 to select video signals from the ceiling camera 103 and select the DVR 106b as the destination and start recording a ceiling camera image in the DVR 106b.

After that, when an endoscopic operation is started on the patient 48 in the generally anesthetized condition, the controller 22 switches off the shadowless lamp 55. As a result, the system controller 22 detects the occurrence of the switch-off (OFF) event from the shadowless lamp 55.

By detecting the event occurrence by the system controller 22, a control trigger signal = {Trigger Flag = 1 (=stop recording), Appliance ID = 02} is transmitted from the system controller 22 to the AV controller 108.

Then, based on the control trigger signal = {Trigger Flag = 1 (=stop recording), Appliance ID = 02}, the AV controller 108 controls the DVR 106b through the recorder I/F section 232 and controls the video signal switcher 151 through the control trigger extracting section 236 to stop recording a ceiling camera image in the DVR 106b.

On the other hand, when the system controller 22 starts controlling the insufflator 14, the system controller 22 detects the occurrence of an event of control start from the insufflator 14.

By detecting the event occurrence by the system controller 22, a control trigger signal = {Trigger Flag = 0 (=start recording), Appliance ID = 00} is transmitted from the system controller 22 to the AV controller 108.

Then, based on the control trigger signal = {Trigger Flag = 0 (=start recording), Appliance ID = 00}, the AV controller 108 controls the DVD 106a through the recorder I/F section 232 and controls the video signal switcher 151 through the control trigger extracting section 236 to select video signals from the endoscope camera device 15 and select the DVD 106a as the destination and start recording an endoscopic image in the DVD 106a.

In order to externally perform a treatment on an affected part, for example, during a technique in progress, the system controller 22 stops controlling the insufflator 14. Thus, the system controller 22 detects the occurrence of the control stop event from the insufflator 14.

By detecting the event occurrence by the system controller 22, a control trigger signal = {Trigger Flag = 1 (=stop recording), Appliance ID = 00} is transmitted from the system controller 22 to the AV controller 108.

Then, based on the control trigger signal = {Trigger Flag = 1 (=stop recording), Appliance ID = 00}, the AV controller 108 controls the DVD 106a through the recorder I/F section 232 and controls the video signal switcher 151 through the control trigger extracting section 236 to stop recording an endoscopic image in the DVD 106a.

On the other hand, in order to externally perform a treatment on an affected part, the system controller 22 switches on the shadowless lamp 55. As a result, the system controller 22 detects the occurrence of the switch-on (ON) event from the shadowless lamp 55.

By detecting the event occurrence by the system controller 22, a control trigger signal = {Trigger Flag = 0 (=start recording), Appliance ID = 02} is transmitted from the system controller 22 to the AV controller 108.

Then, based on the control trigger signal = {Trigger Flag = 0 (=start recording), Appliance ID = 02}, the AV controller 108 controls the DVR 106b through the recorder I/F section 232 and controls the video signal switcher 151 through the control trigger extracting section 236 to select video signals from the ceiling camera 103 and select the DVR 106b as the destination and start recording a ceiling camera image in the DVR 106b.

After that, when the external treatment on the affected part ends and an endoscopic operation is restarted, the system controller 22 switches off the shadowless lamp 55. As a result, the system controller 22 detects the occurrence of the switch-off (OFF) event from the shadowless lamp 55.

By detecting the event occurrence by the system controller 22, a control trigger signal = {Trigger Flag = 1 (=stop recording), Appliance ID = 02} is transmitted from the system controller 22 to the AV controller 108.

Then, based on the control trigger signal = {Trigger Flag = 1 (=stop recording), Appliance ID = 02}, the AV controller 108 controls the DVR 106b through the recorder I/F section 232 and controls the video signal switcher 151 through the control trigger extracting section 236 to stop recording a ceiling camera image in the DVR 106b.

On the other hand, when the system controller 22 restarts controlling the insufflator 14, the system controller 22 detects the occurrence of an event of control start from the insufflator 14.

By detecting the event occurrence by the system controller 22, a control trigger signal = {Trigger Flag = 0 (=start recording), Appliance ID = 00} is transmitted from the system controller 22 to the AV controller 108.

Then, based on the control trigger signal = {Trigger Flag = 0 (=start recording), Appliance ID = 00}, the AV controller 108 controls the DVD 106a through the recorder I/F section 232 and controls the video signal switcher 151 through the control trigger extracting section 236 to select video signals from the endoscope camera device 15 and select the DVD 106a as the destination and start recording an endoscopic image in the DVD 106a.

Then, when the endoscopic operation ends, the system controller 22 stops controlling the insufflator 14. Thus, the system controller 22 detects the occurrence of an event of control stop from the insufflator 14.

By detecting the event occurrence by the system controller 22, a control trigger signal = {Trigger Flag = 1 (=stop recording), Appliance ID = 00} is transmitted from the system controller 22 to the AV controller 108.

Then, based on the control trigger signal = {Trigger Flag = 1 (=stop recording), Appliance ID = 00}, the AV controller 108 controls the DVD 106a through the recorder I/F section 232 and controls the video signal switcher 151 through the control trigger extracting section 236 to stop recording an endoscopic image in the DVD 106a.

On the other hand, in order to perform a stitching-up treatment on the patient 48 after the endoscopic operation ends, the system controller 22 switches on the shadowless lamp 55. As a result, the system controller 22 detects the occurrence of the switch-on (ON) event from the shadowless lamp 55.

By detecting the event occurrence by the system controller 22, a control trigger signal = {Trigger Flag = 0 (=start recording), Appliance ID = 02} is transmitted from the system controller 22 to the AV controller 108.

Then, based on the control trigger signal = {Trigger Flag = 0 (=start recording), Appliance ID = 02}, the AV controller 108 controls the DVR 106b through the recorder I/F section 232 and controls the video signal switcher 151 through the control trigger extracting section 236 to select video signals from the ceiling camera 103 and select the DVR 106b as the destination and start recording a ceiling camera image in the DVR 106b.

After that, when the entire technique ends, the controller 22 switches off the shadow less lamp 55. As a result, the system controller 22 detects the occurrence of the switch-off (OFF) event from the shadowless lamp 55.

By detecting the event occurrence by the system controller 22, a control trigger signal = {Trigger Flag = 1 (=stop recording), Appliance ID = 02} is transmitted from the system controller 22 to the AV controller 108.

Then, based on the control trigger signal = {Trigger Flag = 1 (=stop recording), Appliance ID = 02}, the AV controller 108 controls the DVR 106b through the recorder I/F section 232 and controls the video signal switcher 151 through the control trigger extracting section 236 to stop recording a ceiling camera image in the DVR 106b.

Furthermore, when the system controller 22 stops controlling the anesthetic device 51, the system controller 22 detects the occurrence of the control stop event from the anesthetic device 51.

By detecting the event occurrence by the system controller 22, a control trigger signal = {Trigger Flag = 1 (=stop recording), Appliance ID = 01} is transmitted from the system controller 22 to the AV controller 108.

Then, based on the control trigger signal = {Trigger Flag = 1 (=stop recording), Appliance ID = 01}, the AV controller 108 controls the VTR 106c through the recorder I/F section 232 and controls the video signal switcher 151 through the control trigger extracting section 236 to stop recording a room camera image in the VTR 106c.

In this way, according to the present embodiment, the system controller 22 monitors the control states of the peripheral medical appliances, detects a predetermined change in control states as an event occurrence and generates a control trigger signal. Then, the system controller 22 outputs the control trigger signal to the AV controller 108 by the predetermined protocol, whereby the AV controller 108 indirectly detects the event occurrence based on the control trigger signal. The AV controller 108 controls recording on a recorder, which corresponds to the event occurrence, through the recorder I/F section 232 based on the control trigger signal and controls the video signal switcher 151 through the control trigger extracting section 236 to output video signals of the image corresponding to the event occurrence to the recorder corresponding to the event occurrence.

Thus, according to the present embodiment, when a predetermined change occurs in the control states of medical appliances, recording a desired medical image in a desired recorder can be automatically controlled in accordance with the occurring time. Therefore, an optimum image can be securely recorded in accordance with the progress of an operation.

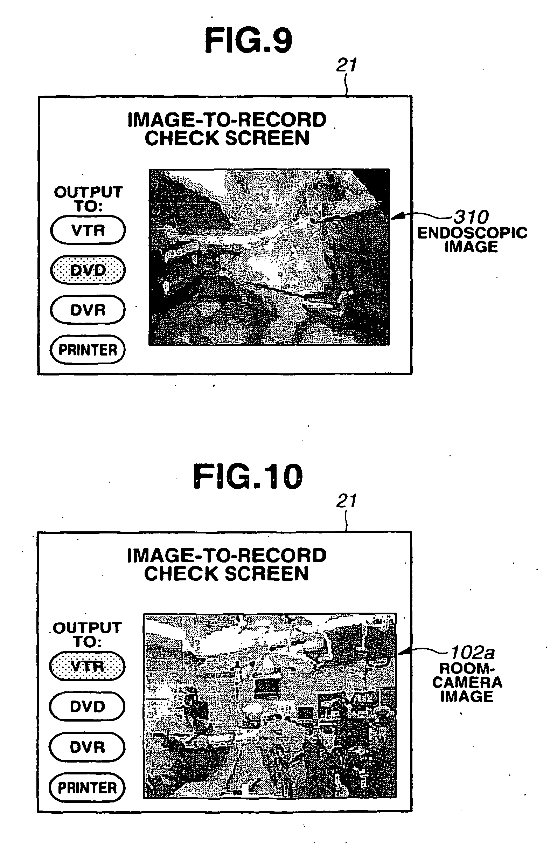

As described above, the image to record in the recorder group 106 of the video signal switcher 151 is also outputted to the touch panel I/F section 224 of the system controller 22 according to the present embodiment (refer to Fig. 3). Then, the system controller 22 can display an image-to-record check screen including an image being recorded and the destination on the operation panel 21, as shown in Figs. 9 and 10, through the touch panel I/F section 224. From the image-to-record check screen, a surgeon can easily and securely check that an endoscopic image 310 is being recorded in the DVD 106a in Fig. 9 and that a room camera image 103a is being recorded in the VTR 106c in Fig. 10, for example.

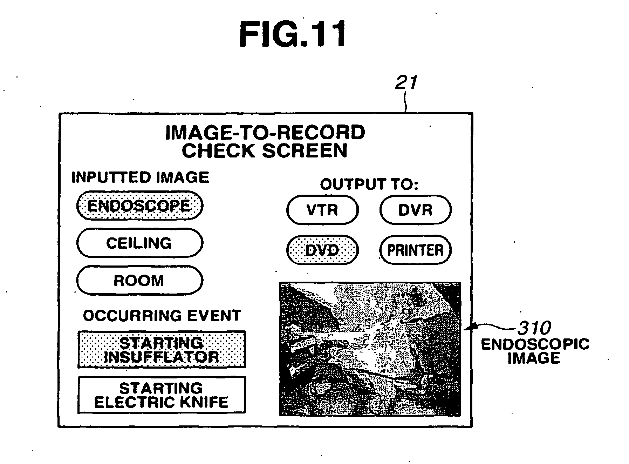

The system controller 22 can further display the image-to-record check screen as shown in Fig. 11 on the operation panel 21 through the touch panel I/F section 224. On the image-to-record check screen in Fig. 11, the image to record can be identified by text information describing the name of the inputted image instead of the image. In Fig. 11, the name of the medical appliance in which an event occurs can be identified by text information. Furthermore, having described that the AV system control section 235 of the AV controller 108 internally stores images to record and recorder data, which are associated with the appliance ID in the control trigger signal, as table data by using the touch panel function of the operation panel 21, the present invention is not limited thereto. The images to record and recorder data on the table data may be updated by changing the image to record and the destination in Fig. 11.

Having described the configuration according to the present embodiment in which the ceiling camera 103 is controlled by the system controller 22, the present invention is not limited thereto. The AV controller 108 may control the ceiling camera 103 instead. Also, having described the configuration in which the shadowless lamp 55 and shadowless lamp camera 58 are also controlled by the system controller 22 according to the present embodiment, the present invention is not limited thereto. The AV controller 108 may control the shadowless lamp 55 and shadowless lamp camera 58 instead.

Furthermore, having described that the video signal switcher 151 controls the output to be recorded in a recorder in the image recorder group 106 based on a control trigger signal from the AV controller 108 according to the present embodiment, a timer may be provided within the video signal switcher 151, and the video signal switcher 151 may start the timer in response to a control command based on a control trigger signal from the AV controller 108 and output for recording in a recorder after a lapse of a predetermined period of time. By delaying the start or stop of recording by using the timer, the illumination of the shadowless lamp may be stabilized before an image of the ceiling camera 103 is recorded.

Having described that the video signal switcher 151 controls the output to be recorded in a recorder in the image recorder group 106 based on a control trigger signal from the AV controller 108, not only the latest moving picture based on a control trigger signal but also a frame image of video signals in five minutes, for example, immediately before the start of the control based on the control trigger signal stored in the video storage section 153 may be recorded in this case. In particular, when a control trigger signal is generated by handling a change in control state of the electric knife device 13 as an event occurrence, the image immediately before the output from the electric knife device is also actually recorded in the recorder group 106. Thus, the images before and after the time when the electric knife device outputs can be checked after the operation.

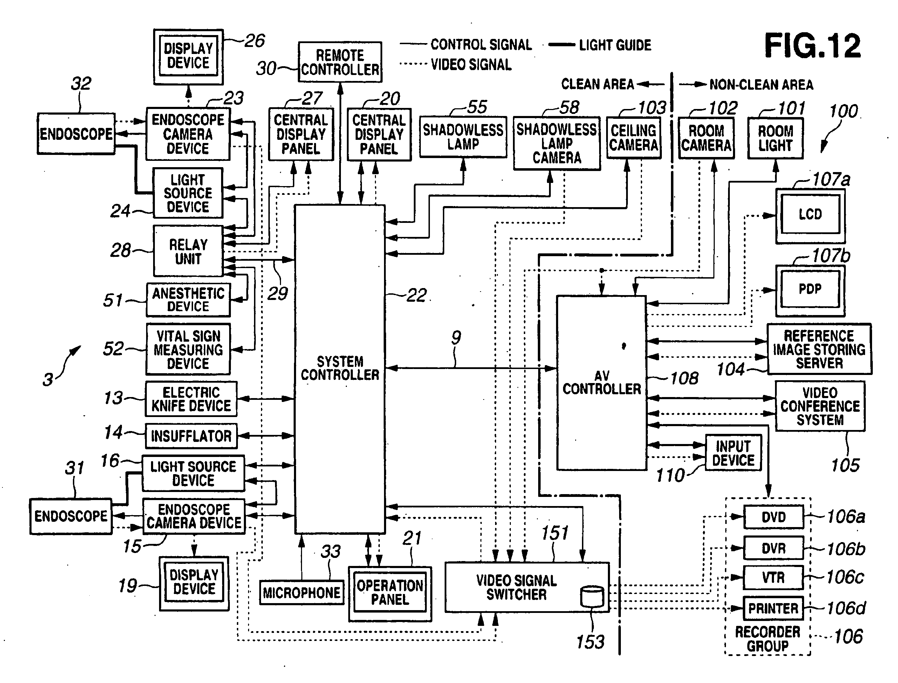

Having described that the video signal switcher 151 is provided within the AV system 100 according to the present embodiment, the present invention is not limited thereto. The video signal switcher 151 may be provided within the endoscopic operation system 3, as shown in Fig. 12.

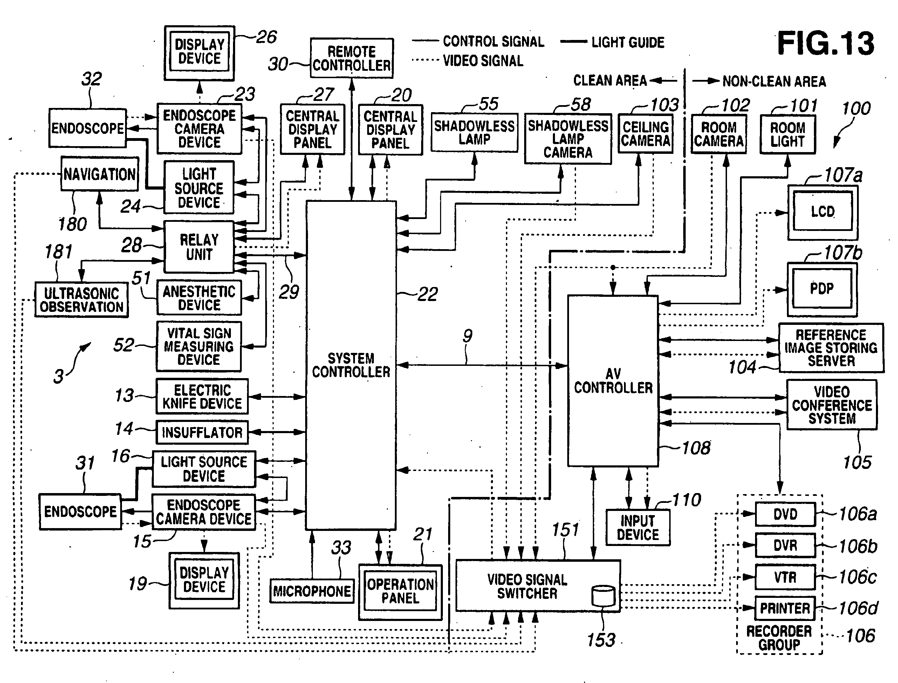

Having described the present embodiment by using the endoscopic images of the endoscopes 31 and 32, for example, the endoscopic operation system 3 may include a navigation device 180 that supports a technique and/or an ultrasonic observation apparatus 181 that performs ultrasonic observation, as shown in Fig. 13. In this case, the system controller 22 handles the navigation device 180 and ultrasonic observation apparatus 181 as appliances in which an event is to occur. Then, by outputting video signals of a support image by the navigation device 180 and video signals of an ultrasonic observation image by the ultrasonic observation apparatus 181 to the video signal switcher 151, the video signals of the support image and the video signals of the ultrasonic observation image may be configured to record as medical images in a recorder within the recorder group 106 upon occurrence of events.

### [Second Embodiment]

Since a second embodiment is mostly identical to the first embodiment, only differences therebetween will be described. The same reference numerals are given to the same components, and the description thereon will be omitted below.

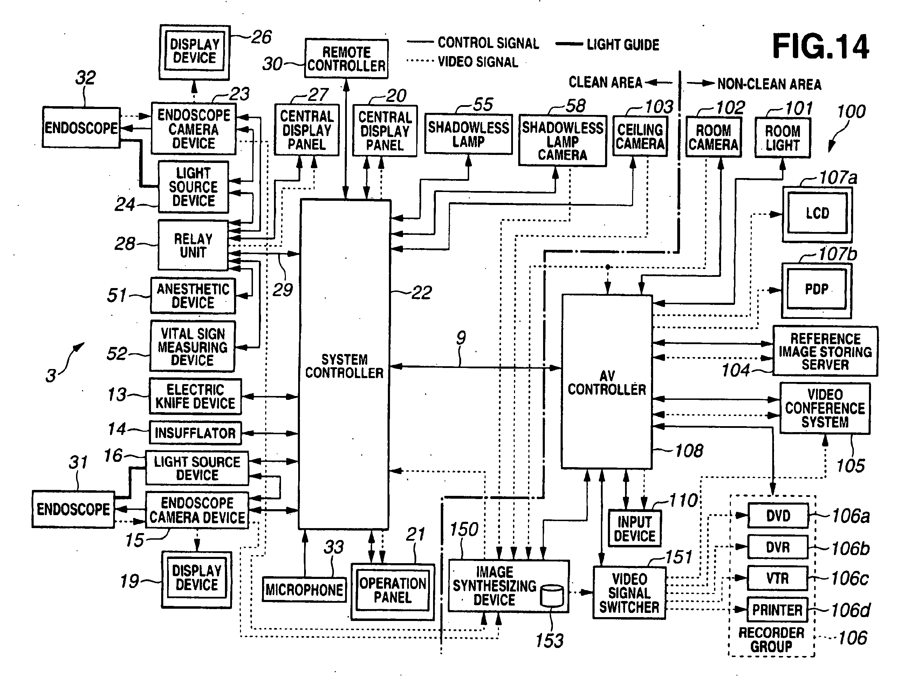

As shown in Fig. 14, the AV system 100 includes an image synthesizing device 150 according to the present embodiment. The image synthesizing device 150 receives the input of video signals of endoscopic images from the endoscope camera devices 15 and 23, video signals from the ceiling camera 103, video signals from the room camera 102, and video signals from the shadowless lamp camera 58, creates a synthesized image from the video signals under the control of the AV controller 108, and outputs the synthesized image to the video signal switcher 151. The image synthesizing device 150 outputs the synthesized image also to the system controller 22. Thus, the system controller 22 can display the synthesized image on the operation panel 21. According to the present embodiment, the video storage section 153 is provided in the image synthesizing device 150.

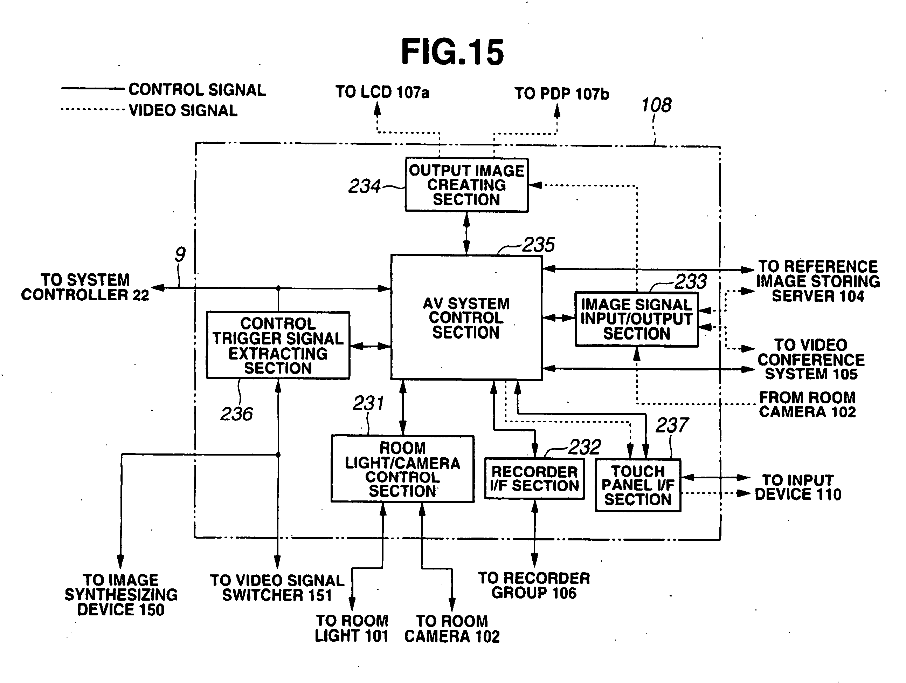

As shown in Fig. 15, the AV controller 108 controls the video signal switcher 151, like the first embodiment, and controls the image synthesizing device 150 through the trigger signal extracting section. The rest of the configuration is identical to that of the first embodiment.

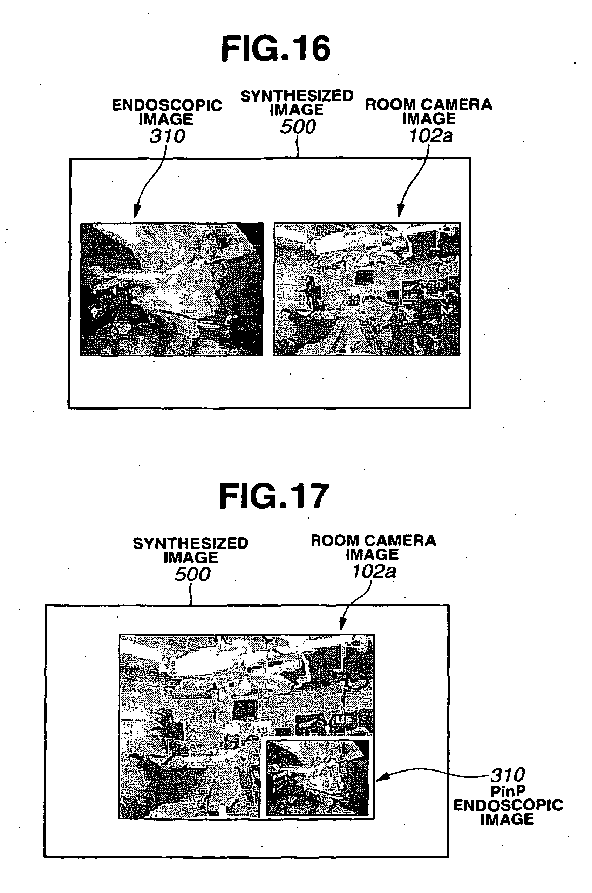

According to the present embodiment having this configuration, the image synthesizing device 150 creates a synthesized image 500 resulting from the synthesis of an endoscopic image 310 and a room camera image 102a in parallel, as shown in Fig. 16, for example, based on a control trigger signal from the AV controller 108 and outputs the synthesized image 500 to the video signal switcher 151. Like the first embodiment, the video signal switcher 151 outputs the video signals of the synthesized image to a predetermined recorder within the recorder group 106 based on a control trigger signal.

Notably, as shown in Fig. 17, the image synthesizing device 150 may create a synthesized image 500 having one image in the other image (Picture-in-Picture: PinP) (that is, synthesized image 500 having the endoscopic image 310 in the room camera image 102a as PinP by handling the room camera image 102a and endoscopic image 310 as parent and child images, respectively) and output the synthesized image 500 to the video signal switcher 151.

Having described that the image synthesizing device 150 creates a synthesized image as shown in Fig. 16 or 17 above, video signals may be passed through and outputted from the image synthesizing device 150 to the video signal switcher 151 without simply synthesized. The synthesis control in the image synthesizing device is performed by the AV system control section 235 based on a control trigger signal.

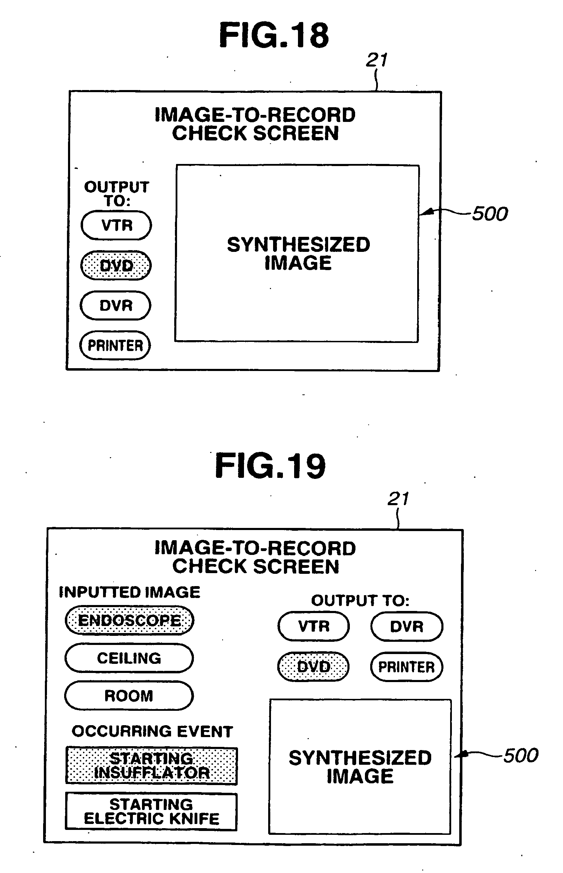

The synthesized image 500 created in this way can be checked as an image to record on the operation panel 21 as described above, as shown in Fig. 18 or 19. Since the details of Figs. 18 and 19 are the same as those of Figs. 9, 10 and 11 according to the first embodiment, the description thereof will be omitted (where Fig. 18 corresponds to Figs. 9 and 10 and Fig. 19 corresponds to Fig. 11). The other operations are the same as those of the first embodiment.

In this way, the present embodiment with the configuration and operations above can also obtain the same effects as those of the first embodiment and can securely record multiple pieces of image information in one recorder in accordance with the progress of an operation by creating a synthesized image from multiple images by the image synthesizing device 150.

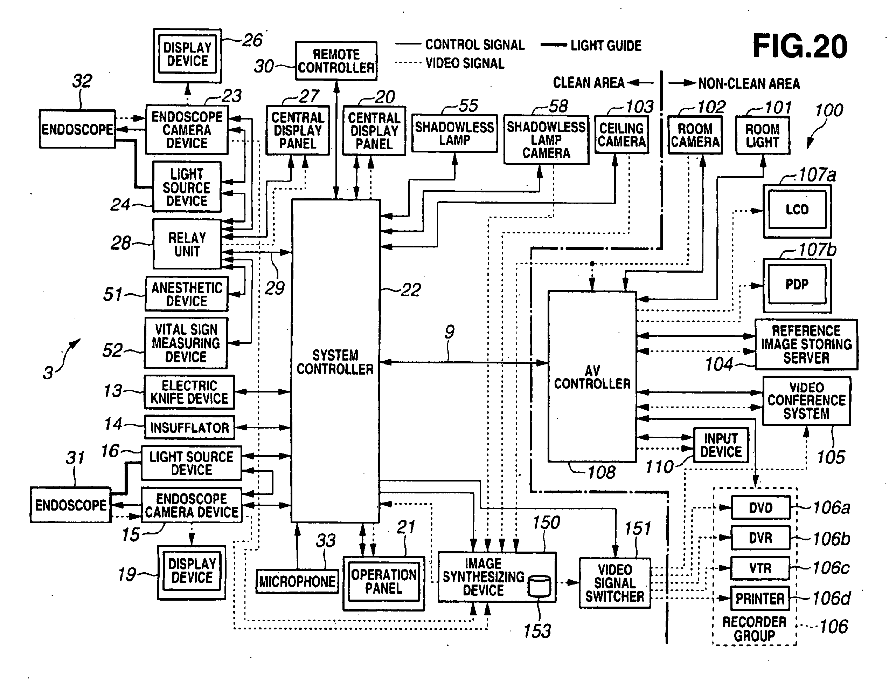

Having described that the image synthesizing device 150 and video signal switcher 151 are provided within the AV system 100 according to the present embodiment, the present invention is not limited thereto. As shown in Fig. 20, the image synthesizing device 150 and video signal switcher 151 may be provided within the endoscopic operation system 3.

### [Third Embodiment]

Since a third embodiment is mostly identical to the second embodiment, only differences therebetween will be described. The same reference numerals are given to the same components, and the description thereon will be omitted below.

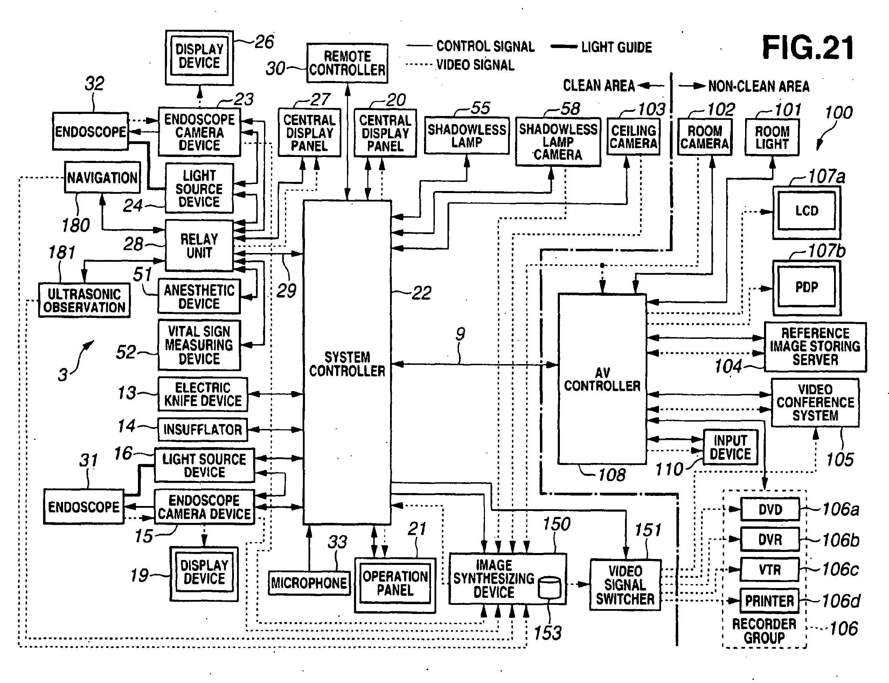

As shown in Fig. 21, the endoscopic operation system 3 includes the navigation device 180 that supports a technique and the ultrasonic observation apparatus 181 that performs ultrasonic observation according to the present embodiment. Video signals of a support image by the navigation device 180 and video signals of an ultrasonic observation image by the ultrasonic observation apparatus 181 are outputted to the image synthesizing device 150.

According to the present embodiment, the video signal switcher 151 is configured to be capable of outputting video signals selectively not only to the recorder group 106 but also to a video conference system. The rest of the configuration is the same as that of the second embodiment.

Also in the present embodiment, like the second embodiment, the image synthesizing device 150 outputs a synthesized image also to the system controller 22.

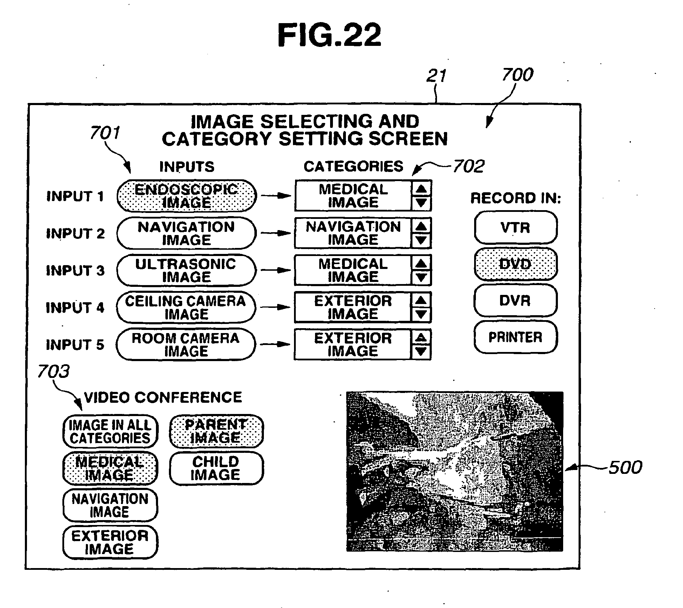

According to the present embodiment, the system controller 22 can display on the operation panel 21 an image selecting and category setting screen 700 having a synthesized image, which is an image-to-record 500, from the image synthesizing device 150, as shown in Fig. 22.

The image selecting and category setting screen 700 has, in addition to the synthesized image and information on the recorder for the synthesized image (destination), an input image selecting area 701 on which an input image inputted to the image synthesizing devise 150 is selected, a category setting area 702 on which an input image is classified under a predetermined category to register, and a video conference output category setting area 703 on which a category to be outputted to the video conference system 105 is selected.

The image selecting and category setting screen 700 allows a surgeon to set an input image inputted to the image synthesizing device 150 under a desired category and register the input image with the system controller 22 by using a touch panel function of the operation panel 21.

For example, if the input image is an endoscopic image, the input image is categorized as a medical image. In the same manner, if the input image is a navigation image, the input image is categorized as a navigation image. If the input image is an ultrasonic image, the input image is categorized as a medical image. If the input image is a ceiling camera image, the input image is categorized as an exterior image. If the input image is a room camera image, the input image is categorized as the exterior image. Then, these categorized images are registered with the system controller 22.

The image selecting and category setting screen 700 also allows a surgeon to set a category for images to be outputted from the image synthesizing device 150 to the video conference system 105 and register the images with the system controller 22 by using a touch panel function of the operation panel 21.

In this way, after the category of the input image and the category of the image to be outputted to the video conference system 105 are set in the system controller 22, the system controller 22 transmits these pieces of information to the AV controller 108 via the cable 9.

The AV controller 108 controls the video signal switcher 151 to only output images under the registered categories to the video conference system 105.

The video conference system 105 is connected to an in-hospital LAN (not shown, and is connected to the terminals in a conference room and a medical office and also can be connected to a terminal in a back-office within a hospital, for example.

For this reason, when the video conference system 105 is connected to a conference room and a medical office, images including all categories may be defined in images to be outputted to the video conference system 105. When the video conference system 105 is connected to a back-office in a hospital, the category for images to be outputted to the video conference system 105 may be limited to the exterior image. Thus, the output of images can be controlled over the in-hospital LAN, keeping the security by managing necessary images only.

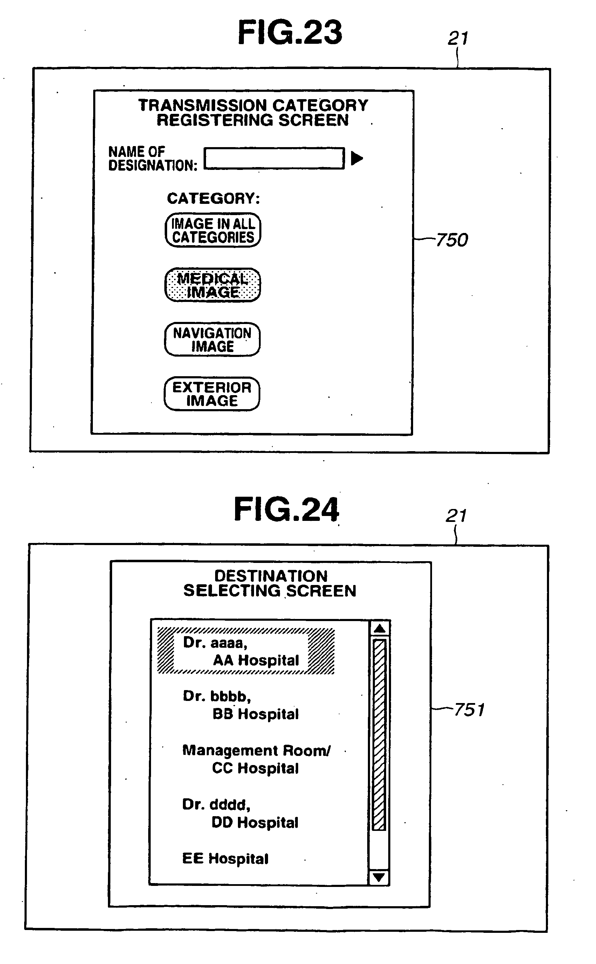

As shown in Fig. 23, the system controller 22 can cause the operation panel 21 to display a transmission category registering screen 750.

As described above, the video conference system 105 is connected to an in-hospital LAN and can connect to an external network through the in-hospital LAN. The transmission category registering screen 750 is a screen for setting the category of an image to be transmitted to a destination connecting to the external network through the video conference system 105.

The transmission category registering screen 750 allows a surgeon to specify the name of a destination and register a transmittable image category with the system controller 22 by using a touch panel function of the operation panel 21.

After transmission category information is registered with the system controller 22 through the transmission category registering screen 750, the system controller 22 can cause the operation panel 21 to display a destination selecting screen 751 as shown in Fig. 24.

The destination selecting screen 751 allows transmitting an image to a selected destination, which connects to the external network through the video conference system 105, by using a touch panel function of the operation panel 21. However, since the transmittable image category is set for each destination through the transmission category registering screen 750, the image can be transmitted by keeping necessary and sufficient security.

It is apparent that the wide variety of embodiments of the present invention can be configured based on the present invention without departing from the scope of the invention. The present invention is not limited to a specific embodiment thereof except for the appended claims.

## Claims

1. An operation system comprising:
a first control system (22) controlling multiple medical appliances (13, 14, 16, 24, 51, 52) and one or more input devices (21, 30) for user input;
a second control system (108) controlling multiple image recording means (106a-d);
appliance monitoring means (226) for monitoring the control states of the multiple medical appliances (13, 14, 16, 24, 51, 52);
multiple image pickup means (15, 23, 55, 102, 103) for generating video signals;
image output means (151) for receiving the input of the video signals from said multiple image pickup means (15, 23, 58, 102, 103) and outputting the video signals selectively to the multiple image recording means (106a-d) according to a control signal;
control signal generating means (227) for generating the control signal that controls the image output means (151); and
image recording control means (235) for controlling the multiple image recording means (106a-d) based on the control signal,
wherein each of the medical appliances (13, 14, 16, 24, 51, 52) is associated with at least one of the image pick-up means (15, 23, 58, 102, 103) and at least one of the image recording means (106a-d);
**characterised in that** the control signal is based on a monitoring result from the appliance monitoring means (226).

2. The operation system according to Claim 1, wherein the first control system (22) has at least the appliance monitoring means (226) and the control signal generating means (227).

3. The operation system according to Claim 1 or 2, further comprising output image display means (107a, 107b) for displaying an image of the video signals outputted by the image output means (151).

4. The operation system according to Claim 3,
wherein the output image display means (107a, 107b) has graphic user interface means; and
the control content of the control signal generated by the control signal generating means (227) is defined through the graphic user interface means.

5. The operation system according to one of claims 1 to 4, wherein the monitoring result from the appliance monitoring means (226) occurs at a timing of start and/or stop of an operation of the medical appliance (13, 14, 16, 24, 51, 52).

6. A technique image recording control method comprising:
an appliance monitoring step of monitoring the control states of multiple medical appliances (13, 14, 16, 24, 51, 52) controlled by a first control system (22) also controlling one or more input devices (21, 30) for user input;
an image output step of receiving the input of video signals from multiple image pickup means (15, 23, 58, 102, 103) and outputting the video signals selectively to multiple image recording steps utilizing multiple image recording means (106a-d) controlled by a second control system (108) based on a control signal;
a control signal generating step of generating the control signal that controls the processing in the image output step, and
an image recording control step of controlling the processing in the multiple image recording steps based on the control signal,
wherein each of the medical appliances (13, 14, 16, 24, 51, 52) is associated with at least one of the image pickup means (15, 23, 58, 102, 103) and at least one of the image recording means (106a-d);
**characterised in that** the control signal is based on a monitoring result from the appliance monitoring step.

## Patentansprüche

1. Ein Betriebssystem, das umfasst:
ein erstes Steuerungssystem (22), das mehrere medizinische Geräte (13, 14, 16, 24, 51, 52) und ein oder mehrere Eingabegeräte (21, 30) für Benutzereingaben steuert,
ein zweites Steuerungssystem (108), das mehrere Bildaufzeichnungsgeräte (106a-d) steuert,
eine Geräteüberwachungsvorrichtung (226) zur Überwachung der Steuerzustände der mehreren medizinischen Geräte (13, 14, 16, 24, 51, 52),
mehrere Bildaufnahmegeräte (15, 23, 58, 102, 103) zur Erzeugung von Videosignalen,
ein Bildausgabegerät (151) für den Empfang des Eingangs der Videosignale von den mehreren Bildaufnahmegeräten (15, 23, 58, 102, 103) und für die selektive Ausgabe der Videosignale an die mehreren Bildaufzeichnungsgeräte (106a-d) gemäß einem Steuersignal,
einen Steuersignalgenerator (227) zur Erzeugung des Steuersignals, das das Bildausgabegerät (151) steuert, und
ein Bildaufzeichnungssteuergerät (235) zur Steuerung der mehreren Bildaufzeichnungsgeräte (106a-d) auf der Grundlage des Steuersignals,
bei dem jedes der mehreren medizinischen Geräte (13, 14, 16, 24, 51, 52) wenigstens einem der Bildaufnahmegeräte (15, 23, 58, 102, 103) und wenigstens einem der Bildaufzeichnungsgeräte (106a-d) zugeordnet ist,
**dadurch gekennzeichnet, dass**
das Steuersignal auf dem Überwachungsergebnis des Anwendungsüberwachungsgeräts (226) beruht.

2. Das Betriebssystem nach Anspruch 1, bei dem das erste Steuersystem (22) wenigstens die Geräteüberwachungsvorrichtung (226) und den Steuersignalgenerator (227) aufweist.

3. Das Betriebssystem nach Anspruch 1 oder 2, das ferner Ausgabebildanzeigegeräte (107a, 107b) für das Anzeigen eines Bildes des von dem Bildausgabegerät (151) ausgegebenen Videosignals aufweist.

4. Das Betriebssystem nach Anspruch 3,
bei dem die Ausgabebildanzeigegeräte (107a, 107b) ein grafische Benutzerschnittstelle aufweisen und
der Steuerinhalt des von dem Steuersignalgenerator (227) erzeugten Steuersignals durch die grafische Benutzerschnittstelle definiert ist.

5. Das Betriebssystem nach einem der Ansprüche 1 bis 4, bei dem das Überwachungsergebnis der Geräteüberwachungsvorrichtung (226) bei der Zeitgebung für Start und/oder Stopp des Einsatzes eines medizinischen Geräts auftritt.

6. Ein Steuerungssystem für die Bildaufzeichnung eines Verfahrensablaufs, das umfasst:
einen Geräteüberwachungsschritt für die Überwachung des Steuerzustandes von mehreren medizinischen Geräten (13, 14, 16, 24, 51, 52), das durch ein erstes Steuerungssystem (22) gesteuert wird, das auch eines oder mehrere Eingabegeräte (21, 30) für Benutzereingaben steuert,
einem Bildausgabeschritt des Empfangens des Eingangs von Videosignalen von mehreren Bildaufnahmegeräten (15, 23, 58, 102, 103) und Ausgebens der Videosignale selektive an mehrere Bildaufzeichnungsschritte durch Einsatz mehrerer Bildaufzeichnungsgeräte (106a-d) unter Steuerung durch ein zweites Steuerungssystem (108) auf der Grundlage eines Steuersignals,
einen Erzeugungsschritt für ein Steuersignal zum Erzeugen des Steuersignals, das das Vorgehen in dem Bildausgabeschritt steuert, und
einen Steuerschritt für die Bildaufzeichnung zum Steuern der Verarbeitung in den mehreren Bildaufzeichnungsschritten auf der Grundlage des Steuersignals,
bei dem jede der medizinischen Geräte (13, 14, 16, 24, 51, 52) wenigstens einem der Bildaufnahmegeräte (15, 23, 58, 102, 103) und wenigstens einem der Bildaufzeichnungsgeräte (106a-d) zugeordnet ist,
**dadurch gekennzeichnet, dass**
das Steuersignal auf dem Überwachungsergebnis eines Geräteüberwachungsschritts beruht.

## Revendications

1. Système de fonctionnement comprenant :
un premier système de commande (22) commandant de multiples appareils médicaux (13, 14, 16, 24, 51, 52) et un ou plusieurs dispositifs d'entrée (21, 30) pour une entrée utilisateur ;
un deuxième système de commande (108) commandant des moyens d'enregistrement d'images multiples (106a-106d) ;
un moyen de contrôle d'appareil (226) destiné à contrôler les états de commande des multiples appareils médicaux (13, 14, 16, 24, 51, 52) ;
des moyens de saisie d'images multiples (15, 23, 58, 102, 103) destinés à générer des signaux vidéo ;
un moyen de sortie d'images (151) destiné à recevoir l'entrée des signaux vidéo provenant desdits moyens de saisie d'images multiples (15, 23, 55, 102, 103) et à délivrer en sortie les signaux vidéo sélectivement vers les moyens d'enregistrement d'images multiples (106a-106d), conformément à un signal de commande ;
un moyen de génération de signal de commande (227) destiné à générer le signal de commande qui commande le moyen de sortie d'images (151) ; et
un moyen de commande d'enregistrement d'images (235) destiné à commander les moyens d'enregistrement d'images multiples (106a-106d) sur la base du signal de commande,
dans lequel chacun des appareils médicaux (13, 14, 16, 24, 51, 52) est associé à au moins l'un des moyens de saisie d'images (15, 23, 58, 102, 103) et à au moins l'un des moyens d'enregistrement d'images (106a-106d),
**caractérisé en ce que**
le signal de commande est basé sur un résultat de contrôle du moyen de contrôle d'appareils (226).

2. Système de fonctionnement selon la revendication 1, dans lequel le premier système de commande (22) comporte au moins le moyen de contrôle (226) et le moyen de génération de signal de commande (227).

3. Système de fonctionnement selon la revendication 1 ou 2, comprenant en outre des moyens d'affichage d'images de sortie (107a, 107b) destinés à afficher une image des signaux vidéo délivrés en sortie par le moyen de sortie d'images (151).

4. Système de fonctionnement selon la revendication 3,
dans lequel les moyens d'affichage d'images de sortie (107a, 107b) comportent un moyen d'interface utilisateur graphique ; et
le contenu de commande du signal de commande généré par le moyen de génération de signal de commande (227) est défini par l'intermédiaire du moyen d'interface utilisateur graphique.

5. Système de fonctionnement selon l'une quelconque des revendications 1 à 4, dans lequel le résultat de contrôle du moyen de contrôle d'appareil (226) se produit au moment du lancement et/ou de l'arrêt du fonctionnement des appareils médicaux (13, 14, 16, 24, 51, 52).

6. Procédé de commande d'enregistrement d'images techniques comprenant :
une étape de contrôle d'appareil consistant à contrôler les états de commande des multiples appareils médicaux (13, 14, 16, 24, 51, 52) commandés par un premier système de commande (22) commandant également un ou plusieurs dispositifs d'entrée (21, 30) pour une entrée utilisateur
une étape de sortie d'images consistant à recevoir l'entrée des signaux vidéo provenant des moyens de saisie d'images multiples (15, 23, 58, 102, 103) et à délivrer en sortie les signaux vidéo sélectivement vers les étapes d'enregistrement d'images multiples utilisant les moyens d'enregistrement d'images multiples (106a-106d) commandés par un deuxième système de commande (108) sur la base d'un signal de commande ;
une étape de génération de signal de commande consistant à générer le signal de commande qui commande le traitement dans l'étape de sortie d'images ; et
une étape de commande d'enregistrement d'images consistant à commander le traitement dans les étapes d'enregistrement d'images multiples sur la base du signal de commande,
dans lequel chacun des appareils médicaux (13, 14, 16, 24, 51, 52) est associé à au moins l'un des moyens de saisie d'images (15, 23, 58, 102, 103) et à au moins l'un des moyens d'enregistrement d'images (106a-106d) ;
**caractérisé en ce que**
le signal de commande est basé sur un résultat de contrôle de l'étape de contrôle d'appareil.
